# EUROPEAN PATENT SPECIFICATION

(11) **EP 4 580 571 B1**
(45) Date of publication and mention of the grant of the patent: **30.09.2026**
(21) Application number: 23772102.2
(22) Date of filing: 29.08.2023
(51) Int. Cl.: A61F 13/49, A61F 13/513, A61F 13/514, A41B 9/12, A41B 17/00

(54) **GARMENT**
KLEIDUNGSSTÜCK
VÊTEMENT

(30) Priority: 30.08.2022 GB 202212519
(43) Date of publication of application: 09.07.2025
(73) Proprietor: Wuka Ltd, Hertfordshire HP1 3AH (GB)
(72) Inventor: SLOCOMBE, David, Tewin AL6 0NU (GB); RAUT, Rubina, Tewin AL6 0NU (GB)
(74) Representative: Greaves Brewster LLP
(86) International application number: PCT/EP2023/073694
(87) International publication number: WO 2024/047053

(56) References cited:
- CN-U- 213 697 518
- GB-A- 2 592 873
- GB-A- 2 607 000
- US-A1- 2018 317 564
- US-A1- 2020 297 556
- US-A1- 2021 100 698
- US-A1- 2021 290 447

## Description

### TECHNICAL FIELD

This invention relates to a reusable menstrual and/or incontinence garment. In particular, the invention relates to a reusable menstrual and/or incontinence garment which is suitable for a range of dress sizes.

### BACKGROUND

Period underwear is absorbent clothing designed to be worn during menstruation. The underwear is washable and reusable, providing an environmentally friendly alternative to conventional disposable menstruation hygiene management products, such a menstrual pads and tampons. Likewise, incontinence underwear is reusable undergarment designed to absorb urine. It provides an environmentally friendly alternative to traditional disposable incontinence products.

Conventional menstruation hygiene management products, such as menstrual pads and tampons are sold in a single size, with different absorbencies. The user buying the menstrual pad or tampon will not be concerned with whether the product will fit, solely whether the product has the correct absorbency. This simplicity of one-size products provides ease of distribution to locations that are only suitable for "universal" product items, such as vending machines.

Period underwear may be provided in eight or more sizes and three or more absorbencies, which would require at least 21 different items to be held in stock. This is a barrier to stocking period underwear as it requires a large amount of retail space, a high cost of maintenance of stock levels and has an impact on cash flow locked up in stock. Thus, conventional period underwear is unsuitable for locations such as vending machines, airports, hotels and convenience shops. Locations where menstrual and/or incontinence products are needed often stock disposable products such as tampons or pads due to these problems.

Reusable menstrual and/or incontinence garments are known from CN 213 697 518 U, GB 2 592 873 A, US 2021/100698 A1, US 2020/297556 A1, US 2021/290447 A1 and US 2018/317564 A1.

### SUMMARY OF THE INVENTION

An aspect of the invention provides a reusable menstrual and/or incontinence garment comprising:
a washable outer sheet comprising a front waist portion, a rear waist portion and a crotch portion extending between the front and rear waist portions, the washable outer sheet having a first end at the front waist portion, a second end at the rear waist portion and opposite sides extending between the first and second ends, the front and rear waist portions being joined to form a waist and leg openings, wherein the outer sheet comprises a high-stretch fabric, which is expandable by the stretch of the fabric by at least 80%;
a washable inner sheet comprising an inner front waist portion, an inner rear waist portion and an inner crotch portion extending between the inner front and rear waist portions, the inner sheet having a first end at the front waist portion, a second end at the rear waist portion and opposite sides extending between the first and second ends, wherein the outer sheet comprises a high-stretch fabric, which is expandable by the stretch of the fabric by at least 80%;
a gusset comprising a washable and reusable absorbent layer configured for the absorption of menstrual fluid and/or urine, wherein the gusset is provided at the inner crotch portion of the inner sheet;
wherein the inner sheet and the outer sheet are connected together at their first ends and second ends respectively.

The use of high stretch fabrics for both the outer and inner sheets enables the underwear garment to fit a range of dress sizes. For example, a single underwear garment may be suitable for sizes XS-L or XL-4XL.

It is important that the gusset is held securely in place for all sizes within the size range of the underwear garment, irrespective of the amount of stretch in the fabric during use. The provision of the gusset on a separate inner sheet provides secure positioning of the gusset as the stretch in the inner sheet is independent of the stretch in the outer sheet.

The inner and outer sheet may be connected together at the gusset at one or more gusset attachment zones. The gusset attachment zones may be located towards the first and second ends of the gusset. The gusset attachment zones may be parallel to the first and/or second gusset ends. The gusset attachments zones may extend the full width of the gusset.

The gusset may be elongate with first and second ends and opposite sides. The gusset may be attached to the inner front waist portion of the inner sheet at the gusset's first end and the inner rear waist portion of the inner sheet at the gusset's second end.

The attachment zones may be configured to form unattached portions at one or both of the first and second ends of the gusset, wherein the gusset is unattached to the outer sheet at the unattached portions.

The distance between the first end and the attachment zone at the first end unattached portion may comprise at least about 1cm, or at least about 2cm, or at least about 3cm, or at least about 4cm, or at least about 5cm, or at least about 6cm, or at least about 7cm. The distance between the second end and the attachment zone at the second end unattached portion may comprise at least about 1cm, or at least about 2cm, or at least about 3cm, or at least about 4cm, or at least about 5cm, or at least about 6cm, or at least about 7cm.

The gusset may be attached to the inner front and rear portions of the inner sheet by any suitable means, such as stitching, adhesive, and thermal bonding.

The gusset may have a minimum width in the range of from about 6cm to about 9cm. This has the advantage of providing enhanced absorption and retention of fluids.

The inner and outer sheets may be only connected together at the waist and the gusset.

In an alternative embodiment, the inner and outer sheets are unattached at the gusset. For example, the inner and outer sheets may be only connected together at the waist.

The sides of the inner front and rear portions of the inner sheet are unattached to the outer sheet. This arrangement enhances the secure positioning of the gusset, as it increases the independent stretching of the outer and inner sheets.

In one embodiment, the inner front waist portion and inner rear waist portion of the inner sheet are not joined together. This arrangement maximises the available lengthwise stretch of the inner sheet. Lengthwise stretch is stretch in the longitudinal direction of the sheet, whereas widthwise stretch is stretch in the transverse direction of the sheet.

The first end of the inner sheet may be attached to the first end of the outer sheet at waist attachment zones, the waist attachment zones being located towards outer edges of the first end of the inner sheet.

The first end of the inner sheet may be unattached to the first end of the washable outer sheet at a central location of the first end of the washable inner sheet, the central location being located between the outer edges. This arrangement reduces the total area of inner sheet connected to the outer sheet and thereby increases the amount of available stretch of the inner sheet.

The first end of the inner sheet may be shaped such that there is a recess at the central location between the outer edges.

The second end of the inner sheet may be attached to the second end of the outer sheet at waist attachment zones, the waist attachment zones being located towards outer edges of the second end of the inner sheet.

The second end of the inner sheet may be unattached to the second end of the outer sheet at a central location of the second end of the inner sheet, the central location being located between the outer edges.

The second end of the inner sheet may be shaped such that there is a recess at the central location between the outer edges. The recess maximises the stretch of the inner sheet.

The recess may comprise a curved shape, for example an arc. The curved shape maximises the stretch of the inner sheet.

The outer sheet and inner sheet comprise high stretch fabrics.

The high stretch fabric may be expanded by the stretch of fabric by at least about least 90%, or at about least 100%, or at about least 110%, or at about least 120%, or at least about 130%, or at least about 140%, or at least about 150%.

The high-stretch fabric may be a four-way stretch fabric. Four-way stretch means that the fabric stretches and recovers both widthwise and lengthwise. The fit of the underwear on the vertical axis changes as the underwear size changes. Therefore, underwear which is a midi brief height on an XS wearer might become a hipster height underwear on a size L. This is because the L size wearer is generally taller than the XS wearer, although not always. The applicants have discovered that this problem can be overcome by the use of four-way high stretch fabric to allow the fabric to stretch on the vertical axis as well as stretching in the horizontal axis to adjust the waist size.

Suitable high stretch fabrics for the outer sheet and/or the inner sheet include nylon, polyester, polyamide, elastane. Recycled fabrics, such as recycled nylon may be used.

The high-stretch fabric may comprise elastane. Elastance comprises polyether-polyurea copolymer and is also known as Lycra^{™} and Spandex^{™}. The high-stretch fabric may comprise 15-40% elastance.

In one example, the outer sheet comprises 82% nylon and 18% elastance. In another example, the outer sheet comprises 64% polyamide and 36% elastane. In one example, the inner sheet comprises 82% nylon and 18% elastance. In another example, the inner sheet comprises 64% polyamide and 36% elastane. The inner sheet may comprise the same or different fabric as the outer sheet.

The outer sheet may be seamless. For example, it may be manufactured by a circular knitting machine. A seamless outer sheet will provide an invisible panty line. Additionally, the absence of seams maximises the available stretch of the outer sheet.

The gusset may comprise an inner layer, which in use lies next to the skin, an outer layer and middle layers between the inner and outer layers. Each of the inner, outer and middle layers are washable and reusable. The inner layer may be configured to wick moisture away from the body. The outer layer may be water-impermeable to prevent fluid from leaking. The middle layers are super-absorbent layers which both absorb and retain fluid.

Suitable fabrics for the inner layer include woven or knitted textile material, such as bamboo, cotton or polyester. Suitable fabrics for the middle layer include bamboo, cotton, merino wool or synthetic fibres. The middle layer may comprise felts, woven, knitted brushed textile materials and/or non-woven fabrics. The knitted brushed textile material may comprise one or more of cotton, polyester, viscose, and polypropylene.

Suitable fabrics for the liquid impermeable fabric include thermoplastic polyurethanes (TPU). The outer layer may comprise a non-woven fabric.

Throughout the description and claims of this specification, the words "comprise" and "contain" and variations of the words, for example "comprising" and "comprises", mean "including but not limited to", and do not exclude other components, integers or steps. Moreover the singular encompasses the plural unless the context otherwise requires: in particular, where the indefinite article is used, the specification is to be understood as contemplating plurality as well as singularity, unless the context requires otherwise.

Preferred features of each aspect of the invention may be as described in connection with any of the other aspects.

The invention is defined by the appended claims.

### BRIEF DESCRIPTION OF THE DRAWINGS

One or more embodiments of the invention will now be described, by way of example only, with reference to the accompanying drawings, in which:
Figure 1A is plan view of an outer sheet of an underwear garment, according to a first embodiment;
Figure 1B is a plan view of an inner sheet of an underwear garment, according to the first embodiment;
Figure 1C is a front view of an underwear garment, according to the first embodiment;
Figure 2 is a plan view of a gusset on the inner sheet of an underwear garment, according to the first embodiment;
Figure 3 is a perspective view of the inner and outer sheets of the underwear garment, according to the first embodiment;
Figure 4A is a plan view of the underwear garment according to the first embodiment in an unstretched state;
Figure 4B is a plan view of the underwear garment according to the first embodiment in a stretched state;
Figure 5A is a front view of an underwear garment, according to a second embodiment; and
Figure 5B is a plan view of an underwear garment, according to the second embodiment.

### DETAILED DESCRIPTION

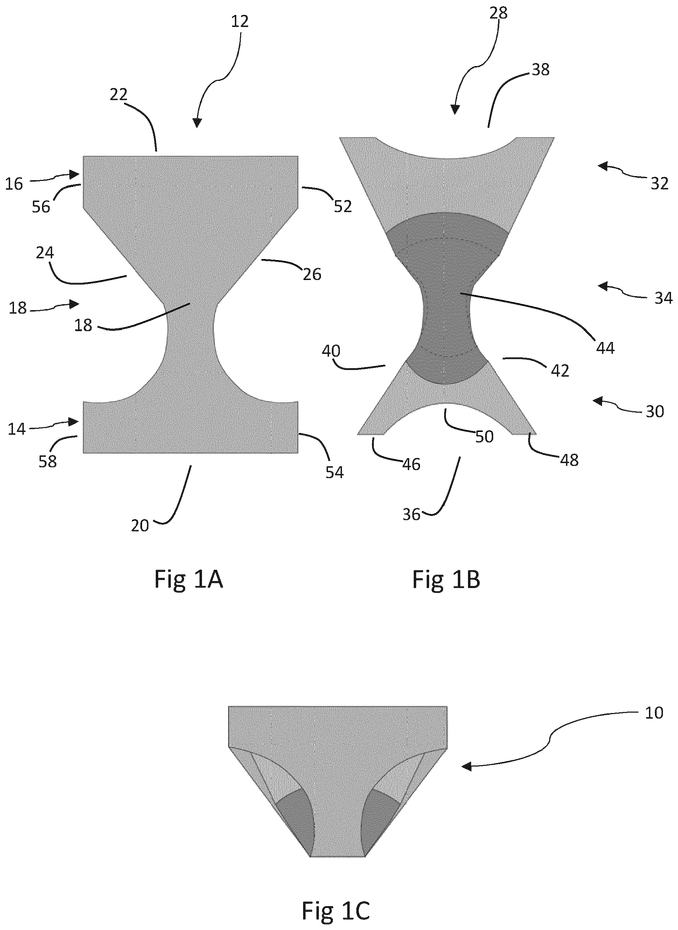

An underwear garment according to a first embodiment is illustrated in Figures 1A,1B and 1C.

The garment 10 has a washable outer sheet 12 illustrated in Fig 1A and a washable inner sheet 28 illustrated in Fig 1B. The assembled garment is illustrated in Fig 1C.

The outer sheet 12 has a front waist portion 14, a rear waist portion 16 and a crotch portion 18 extending between the front and rear waist portions. The outer sheet 12 has a first end 20 at the front waist portion, a second end 22 at the rear waist portion, and first and second sides 24,26 extending between the first and second ends.

The washable inner sheet 28 has an inner front waist portion 30, an inner rear waist portion 32 and an inner crotch portion 34 extending between the inner front and rear waist portions. The inner sheet has a first end 36 at the inner front waist portion, a second end 38 at the inner rear waist portion and first and second sides 40,42 extending between the first and second ends.

A gusset 44 is provided at the inner crotch portion 34 of the inner sheet 28. The gusset includes a washable and reusable absorbent layer configured to absorb menstrual fluid and/or urine. The gusset is attached to the inner front and rear waist portions by any suitable method, such as stitching, adhesive or thermal bonding.

The inner sheet 28 is attached to the outer sheet 12 at their respective first ends 20,36, their respective second ends 22,38 and the gusset 44. In this embodiment, the first end 36 of inner sheet 28 is attached to the first end 20 of outer sheet 12 at waist attachment zones 46, 48 at the outer edges of the first end 36 of the inner sheet 28. A recess 50 is provided between waist attachment zones 46,48 and is unattached to the outer sheet 12. In this embodiment, the recess 50 is arc-shaped but other shaped may be used. A similar arrangement is provided at the second ends 22,38 of the inner sheet 28 and outer sheet 22. The inner sheet 28 is unattached to the outer sheet 12 at its sides 40,42.

The front and rear waist portions 14, 16 of outer sheet 12 are joined together to form a continuous waist, although this is not shown in Fig 1A for clarity. Front wait portion 14 is joined to rear waist portion 16 at edge portions 52,54 on side 26 and at edge portions 54, 56 on side 24. The front and rear waist portions may be joined by seams. Alternatively, the join may be seamless, for example the outer sheet may be manufactured by a circular knitting machine. The first and second ends 20,22 of the outer sheet 12 define the waist of the garment, whilst leg openings are defined by first and second sides 24,26 respectively in the region of the crotch portion 18.

The inner front and rear waist portions 30,32 of inner sheet 28 are not joined together. Therefore, no continuous waist is formed by the inner sheet 28. Instead, the inner sheet 28 hangs from the outer sheet 12 like a hammock. The only attachment between the inner sheet 28 and the outer sheet 12 is at the ends of the inner and outer sheets and at the gusset.

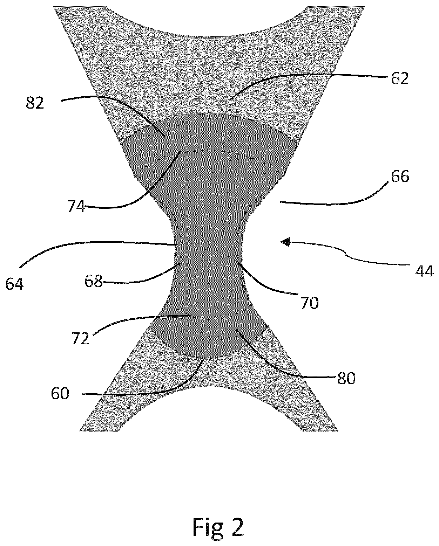

Figure 2 shows the gusset 44 of the inner sheet 28. The gusset includes inner, middle and outer layers, with the inner layer facing the user's skin and the outer layer facing the inner sheet 28. The inner layer is configured to wick moisture from the body, the middle layer includes super absorbent layers to absorb and retain fluid, and the outer layer is water-impermeable to prevent fluid from leaking.

The gusset 44 is elongate and has first and second ends 60,62 and opposite sides 64,66. The gusset 44 is attached to the inner front and rear portions 30,32 at its first and second ends 60,62. Alternatively, the gusset may be attached to the inner crotch portion of the inner sheet.

The gusset 44 is attached to the outer sheet at gusset attachment zones located towards the first and second ends 60,62 and towards the opposite sides 64,66. In this embodiment, the gusset attachment zones are formed by stitching but alternative methods, such as adhesive or thermal bonding may be used. At the opposite sides 64,66, the gusset attachment zones are positioned to leave a small seam allowance between the stitching 68,70 and the sides 64,66, for example 1-5mm. At the first and second ends 60,62, the gusset attachment zones are positioned to leave a larger seam allowance between the stitching 72,74 and ends 60,62, forming unattached portions 76,78. For example the larger seam allowance between stitching 72,74 and ends 60,62 may be 1-7cm. Unattached portions 76,78 may have the same or different dimensions.

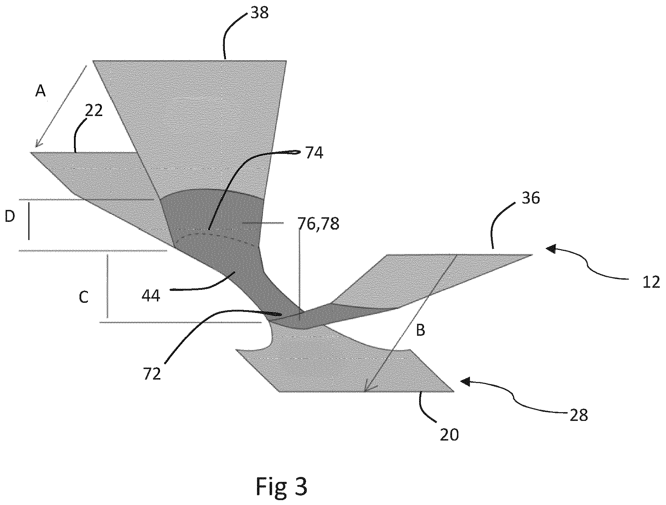

Figure 3 shows the inner and outer sheets 12,28 at a skew angle to illustrate the relationship between the gusset 44, inner sheet 12 and outer sheet 28.

Although not shown for clarity, end 38 of inner sheet is attached to end 22 of outer sheet at A and end 36 of inner sheet is attached to end 20 of outer sheet at B. As discussed with reference to Figs 1A-1C, the inner and outer sheet may not be continuously attached along their ends.

In this embodiment, the inner and outer sheets are attached together at the gusset 44 along attachment zones formed, for example, by stitching 72,74.

This arrangement forms a central zone C and end zones D of the gusset. In the central zone C, the inner and outer sheets are attached together. This arrangement keeps the absorbent area in the correct place.

The gusset is attached to the inner front and rear waist portions of the inner sheet at end zones D but is not attached to the outer sheet in these zones. The gusset in end zones D does not stretch significantly, due to the fabric of the gusset.

The unattached portions in zone D form a "floating gusset" which has several advantages. The portions in zones D (the floating gusset) increases the absorbent area of the gusset. Furthermore, portions in zones D are kept in tension by the inner sheet fabric stretching and pulling it when the underwear is worn. As the gusset is not stitched to the outer sheet in zones D, this provides an advantage that the high stretch fabric in the region of the floating gusset is not constrained from stetching. In contrast, the ability of the outer sheet to stretch is lost in the region of zone C, where it is stitched to the gusset.

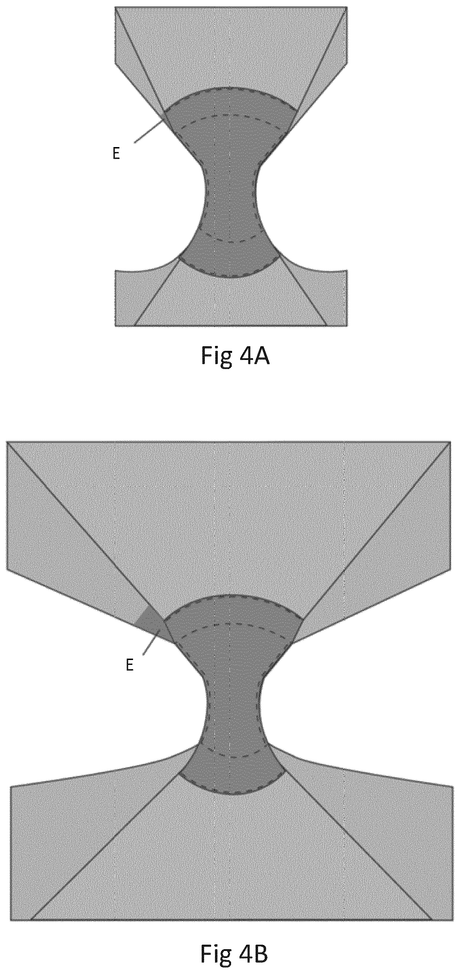

Figures 4A and 4B are a schematic plan views of the garment in unstretched (and unworn) and stretched states respectively. The garments are shown as if cut open at the sides, for clarity.

In both the unstretched and stretched states, the size of the gusset remains substantially the same. However, Fig 4B shows that both the outer sheet and the inner front and rear waist portions of the inner sheet are stretched significantly in the stretched state.

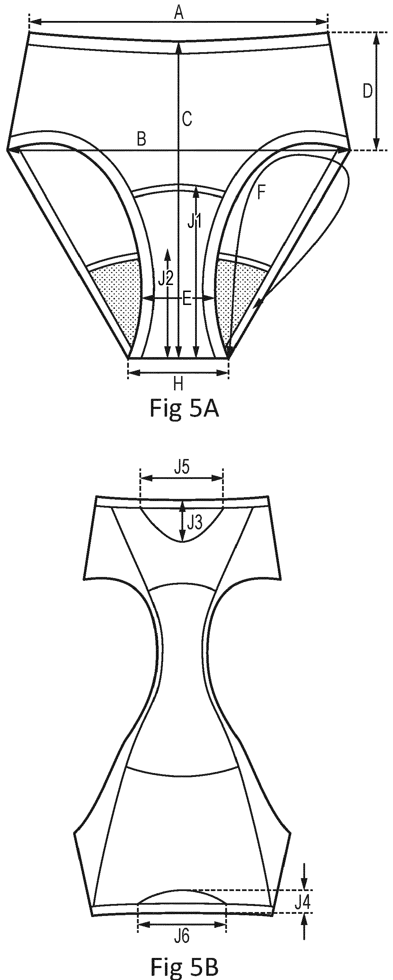

Area E of the outer sheet is labelled in both Figures 4A and 4B. In Figure 4A, the outer sheet is not stretched and area E sits behind the floating gusset. In Figure 4B, the outer sheet is stetched and area E has stretched both lengthwise and widthwise, so that it no longer sits behind the floating gusset. This feature enables the garment to sufficiently to fit four sizes, without compromising gusset security. Figures 5A and 5B show front and plan views of a second embodiment of the underwear garment. This embodiment has similar features to those of the first embodiment. The front and rear waist portions of the outer sheet are shown unattached to each other in the plan view for clarity. The dimensions of the garment of Figs 5A and 5B are provided in Table 1 below.

**Table 1**

| **REFERENCE** | **FEATURE** | **SIZE XS-L (CM)** | **SIZE XL-4XL (CM)** | **TOLERANCE +/- (CM)** |
|---|---|---|---|---|
| A | ½ waist | 24 | 31 | 1 |
| B | ½ hip | 29.5 | 35.5 | 1 |
| C | Mid rise | 29 | 33 | 1 |
| D | Side length | 12 | 12 | 0.5 |
| E | Narrowest of front gusset | 7 | 7.2 | 0.5 |
| F | ½ leg opening (folded) | 23.1 | 28.2 | 1 |
| H | Width of bottom | 10 | 10 | 0.6 |
| J1 | Front gusset height | 17 | 18 | 0.6 |
| J2 | Back gusset height | 9.5 | 10 | 0.6 |
| J3 | Depth of front cut (internal floating) | 6 | 8.4 | 0.5 |
| J4 | Depth of back cut (internal floating) | 3.2 | 5.5 | 0.5 |
| J5 | Width of front cut (internal floating) | 11.5 | 17.8 | 0.6 |
| J6 | Width of back cut (internal floating) | 10.7 | 15.8 | 0.6 |
| P | Width of waist tape | 1.2 | 1.5 | / |

Table 1 gives the dimensions of two sizes of underwear garment. The first size is XS-L, covering XS, S, M, L and the second size is XL-4XL, covering XL, 2XL, 3XL and 4XL. Features A-P of the underwear garment are labelled on Figures 5A and 5B. Features A-P are measured with the garment laid flat on a surface, as illustrated in Fig 5A. All measurements are taken with the fabric unstretched.

Different sized underwear not only has different side waists but also different sizes of other features, for example mid-rise (C) and ½ leg opening (J). The mid-rise C must be selected so that it is appropriate for the full range of sizes accommodated by the underwear, so that the waists sits higher on a smaller body and lower on a larger body without becoming uncomfortable, loose or revealing for either. Likewise, the leg openings J must be dimensioned to be suitable for the full range of sizes. The combined dimensions ensure the underwear stays close to the body.

In the embodiment of Figs 5A,5B, the outer sheet has a composition of 82% nylon, 18% elastane and the inner sheet has a composition of 80% polyester, 20% nylon. The inner layer of the gusset has a composition of 95% cotton and 5% elastane.

Although Figures 5A and 5B illustrate a high-leg brief, this invention is suitable for other underwear garment types, such as brief, high waist, boxer style, bikini.

The inner and outer sheets may be made from fine material, such as nylon or polyamide. Garments made from these fine materials have a low volume when folded, which is advantageous for vending machines.

The high stretch of the menstrual and/or incontinence garment enables a single sized garment to fit multiple dress sizes, whilst still ensuring a good fit. A further benefit is that if the wearer is between sizes or experiences bloating during their period the underwear can be made to fit their body rather than the body fitting a predefined size.

The underwear garment of the present invention has the advantage that a single garment can be adjusted to fit several dress sizes. As illustrated in Table 1, a single garment is suitable for four dress sizes (e.g. XS-L and XL-4XL). However, the garment may be dimensions to fit a different range of sizes, e.g. M-2XL.

The high stretch of the outer sheet is optimised by the use of a "floating gusset", thereby reducing the area in which the gusset is attached to the outer sheet. Additionally, the available stretch is maximised by only attaching the inner sheet to the outer sheet at the ends and gusset.

The high stretch of the inner sheet is optimised by reducing the area of the waist attachment zones by creating a region of non-attachment between waist attachment zones. .It is important that the gusset is held securely in position for all body sizes, irrespective of stretch in the garment. The design of the garment enables it to absorb and hold period flow and/or urine for these different body sizes without leakage from sides, front or back during use. This is due to the secure positioning of the gusset.

The underwear garment of the present invention provides secure positioning of the gusset by several features. The gusset is mounted on the inner sheet, which is separate from the outer sheet and therefore the position of the gusset is not affected by stretching of the outer sheet. Unattached portions at the ends of the gusset are pushed towards the body by the inner sheet, thereby creating a secure fit.

## Claims

1. A reusable menstrual and/or incontinence garment comprising:
a washable outer sheet (12) comprising a front waist portion (14), a rear waist portion (16) and a crotch portion (18) extending between the front and rear waist portions (14,16), the washable outer sheet (12) having a first end (20) at the front waist portion (14), a second end (22) at the rear waist portion (16) and opposite sides (24,26) extending between the first and second ends (20,22), the front and rear waist portions (14,16) being joined to form a waist and leg openings, wherein the outer sheet (12) comprises a high-stretch fabric which is expandable by the stretch of the fabric by at least 80%;
a washable inner sheet (28) comprising an inner front waist portion (30), an inner rear waist portion (32) and an inner crotch portion (34) extending between the inner front and rear waist portions (30,32), the inner sheet (28) having a first end (36) at the front waist portion, a second end (38) at the rear waist portion and opposite sides (40,42) extending between the first and second ends (36,38), wherein the inner front and rear waist portions comprise a high-stretch fabric which is expandable by the stretch of the fabric by at least 80%;
a gusset (44) comprising a washable and reusable absorbent layer configured for the absorption of menstrual fluid and/or urine, wherein the gusset is provided at the inner crotch portion (34) of the inner sheet (28);
wherein the inner sheet (28) and the outer sheet (12) are connected together at their first ends (20,36) and second ends (22,38) respectively.

2. A reusable menstrual and/or incontinence garment according to any preceding claim, wherein the inner and outer sheet (28,12) are connected together at the gusset (44) at one or more gusset attachment zones.

3. A reusable menstrual and/or incontinence garment according to claim 2, wherein the gusset (44) is elongate with first and second ends (60,62) and opposite sides (64,66), wherein attachment zones are configured to form unattached portions (76,78) at one or both of the first and second ends (60,62) of the gusset (44), wherein the gusset (44) is unattached to the outer sheet (12) at the unattached portions (76,78); optionally
wherein the distance between the first end (60) and the attachment zone at the first end unattached portion comprises at least about 1cm, or at least about 2cm, or at least about 3cm, or at least about 4cm, or at least about 5cm, or at least about 6cm, or at least about 7cm and/or wherein the distance between the second end (62)and the attachment zone at the second end unattached portion comprises at least about 1cm, or at least about 2cm, or at least about 3cm, or at least about 4cm, or at least about 5cm, or at least about 6cm, or at least about 7cm.

4. A reusable menstrual and/or incontinence garment according to any preceding claim,
wherein the gusset (44) is attached to the inner front and rear portions of the inner sheet (28) by any suitable means, such as stitching, adhesive, and thermal bonding.

5. A reusable menstrual and/or incontinence garment according to any preceding claim,
wherein the inner and outer sheets (28,12) are only connected together at the waist and the gusset (44).

6. A reusable menstrual and/or incontinence garment according to any preceding claim,
wherein the sides (40,42) of the inner front and rear portions of the inner sheet (28) are unattached to the outer sheet (12).

7. A reusable menstrual and/or incontinence garment according to any preceding claim
wherein the inner front waist portion (30) and inner rear waist portion (32) of the inner sheet (28) are not joined together.

8. A reusable menstrual and/or incontinence garment according to any preceding claim,
wherein the first end (36) of the inner sheet (28) is attached to the first end (20) of the outer sheet (12) at waist attachment zones, the waist attachment zones being located towards outer edges of the first end (36) of the inner sheet (28).

9. A reusable menstrual and/or incontinence garment according to claim 8 wherein the first end (36) of the inner sheet (28) is unattached to the first end (20) of the outer sheet (12) at a central location of the first end (36) of the inner sheet (28), the central location being located between the outer edges.

10. An reusable menstrual and/or incontinence garment according to claim 9, wherein the first end (36) of the inner sheet (28) is shaped such that there is a recess (50) at the central location between the outer edges, optionally wherein the recess (50) comprises a curved shape, optionally an arc.

11. A reusable menstrual and/or incontinence garment according to any preceding claim, wherein the second end (38) of the inner sheet (28) is attached to the second end (22) of the outer sheet (12) at waist attachment zones located towards outer edges of the second end (38) of the inner sheet (28).

12. A reusable menstrual and/or incontinence garment according to claim 11, wherein the second end (38) of the inner sheet (28) is unattached to the second end (22) of the outer sheet (12) at a central location of the second end (38) of the inner sheet (28), the central location being located between the outer edges, optionally wherein the second end (38) of the inner sheet (28) is shaped such that there is a recess (50) at the central location between the outer edges, and optionally wherein the recess (50) comprises a curved shape, optionally an arc.

13. A reusable menstrual and/or incontinence garment according to any preceding claim, wherein the high stretch fabric may be expanded by the stretch of fabric by at least about 90%, or at least about 100%, or at least about 110%, or at least about 120%, or at least about 130%, or at least about 140%, or at least about 150%.

14. A reusable menstrual and/or incontinence garment according to any preceding claim, wherein the high-stretch fabric comprises a four-way stretch fabric and/or wherein the high-stretch fabric is selected from nylon, polyester, polyamide, elastane, optionally wherein the high-stretch fabric comprises 15-40% elastane.

15. A reusable menstrual and/or incontinence garment according to any preceding claim, wherein the outer sheet (12) is seamless.

## Patentansprüche

1. Wiederverwendbares Menstruations- und/oder Inkontinenzkleidungsstück, umfassend:
eine waschbare äußere Lage (12), die einen vorderen Taillenabschnitt (14), einen hinteren Taillenabschnitt (16) und einen Schrittabschnitt (18) umfasst, der sich zwischen dem vorderen und dem hinteren Taillenabschnitt (14,16) erstreckt, wobei die waschbare äußere Lage (12) ein erstes Ende (20) an dem vorderen Taillenabschnitt (14), ein zweites Ende (22) an dem hinteren Taillenabschnitt (16) und gegenüberliegende Seiten (24,26) aufweist, die sich zwischen dem ersten und dem zweiten Ende (20,22) erstrecken, wobei der vordere und der hintere Taillenabschnitt (14,16) zusammengefügt sind, um eine Taillenöffnung und Beinöffnungen auszubilden, wobei die äußere Lage (12) ein hochelastisches Gewebe umfasst, das durch die Elastizität des Gewebes um mindestens 80 % dehnbar ist;
eine waschbare innere Lage (28), die einen inneren vorderen Taillenabschnitt (30), einen inneren hinteren Taillenabschnitt (32) und einen inneren Schrittabschnitt (34) umfasst, der sich zwischen dem inneren vorderen und dem inneren hinteren Taillenabschnitt (30,32) erstreckt, wobei die innere Lage (28) ein erstes Ende (36) an dem vorderen Taillenabschnitt, ein zweites Ende (38) an dem hinteren Taillenabschnitt und gegenüberliegende Seiten (40,42) aufweist, die sich zwischen dem ersten und dem zweiten Ende (36,38) erstrecken, wobei der innere vordere und der innere hintere Taillenabschnitt ein hochelastisches Gewebe umfassen, das durch die Elastizität des Gewebes um mindestens 80 % dehnbar ist;
einen Zwickel (44), der eine waschbare und wiederverwendbare absorbierende Schicht umfasst, die für die Absorption von Menstruationsflüssigkeit und/oder Urin konfiguriert ist, wobei der Zwickel an dem inneren Schrittabschnitt (34) der inneren Lage (28) bereitgestellt ist;
wobei die innere Lage (28) und die äußere Lage (12) an ihren ersten Enden (20,36) beziehungsweise zweiten Enden (22,38) miteinander verbunden sind.

2. Wiederverwendbares Menstruations- und/oder Inkontinenzkleidungsstück nach einem vorhergehenden Anspruch, wobei die innere und die äußere Lage (28,12) an dem Zwickel (44) an einer oder mehreren Zwickelbefestigungszonen miteinander verbunden sind.

3. Wiederverwendbares Menstruations- und/oder Inkontinenzkleidungsstück nach Anspruch 2, wobei der Zwickel (44) länglich ist, mit einem ersten und einem zweiten Ende (60,62) und gegenüberliegenden Seiten (64,66), wobei Befestigungszonen dazu konfiguriert sind, unbefestigte Abschnitte (76,78) an einem oder beiden von dem ersten und dem zweiten Ende (60,62) des Zwickels (44) auszubilden, wobei der Zwickel (44) an den unbefestigten Abschnitten (76,78) nicht an der äußeren Lage (12) befestigt ist; optional
wobei der Abstand zwischen dem ersten Ende (60) und der Befestigungszone an dem unbefestigten Abschnitt des ersten Endes mindestens etwa 1 cm oder mindestens etwa 2 cm oder mindestens etwa 3 cm oder mindestens etwa 4 cm oder mindestens etwa 5 cm oder mindestens etwa 6 cm oder mindestens etwa 7 cm umfasst und/oder wobei der Abstand zwischen dem zweiten Ende (62) und der Befestigungszone an dem unbefestigten Abschnitt des zweiten Endes mindestens etwa 1 cm oder mindestens etwa 2 cm oder mindestens etwa 3 cm oder mindestens etwa 4 cm oder mindestens etwa 5 cm oder mindestens etwa 6 cm oder mindestens etwa 7 cm umfasst.

4. Wiederverwendbares Menstruations- und/oder Inkontinenzkleidungsstück nach einem vorhergehenden Anspruch, wobei der Zwickel (44) durch ein beliebiges geeignetes Mittel, wie etwa Nähen, Kleben und thermisches Binden, an dem inneren vorderen und inneren hinteren Abschnitt der inneren Lage (28) befestigt ist.

5. Wiederverwendbares Menstruations- und/oder Inkontinenzkleidungsstück nach einem vorhergehenden Anspruch, wobei die innere und die äußere Lage (28,12) nur an der Taille und dem Zwickel (44) miteinander verbunden sind.

6. Wiederverwendbares Menstruations- und/oder Inkontinenzkleidungsstück nach einem vorhergehenden Anspruch, wobei die Seiten (40,42) des inneren vorderen und des inneren hinteren Abschnitts der inneren Lage (28) nicht an der äußeren Lage (12) befestigt sind.

7. Wiederverwendbares Menstruations- und/oder Inkontinenzkleidungsstück nach einem vorhergehenden Anspruch, wobei der innere vordere Taillenabschnitt (30) und der innere hintere Taillenabschnitt (32) der inneren Lage (28) nicht miteinander zusammengefügt sind.

8. Wiederverwendbares Menstruations- und/oder Inkontinenzkleidungsstück nach einem vorhergehenden Anspruch, wobei das erste Ende (36) der inneren Lage (28) an dem ersten Ende (20) der äußeren Lage (12) an Taillenbefestigungszonen befestigt ist, wobei sich die Taillenbefestigungszonen zu äußeren Kanten des ersten Endes (36) der inneren Lage (28) hin befinden.

9. Wiederverwendbares Menstruations- und/oder Inkontinenzkleidungsstück nach Anspruch 8, wobei das erste Ende (36) der inneren Lage (28) an einer zentralen Stelle des ersten Endes (36) der inneren Lage (28) nicht an dem ersten Ende (20) der äußeren Lage (12) befestigt ist, wobei sich die zentrale Stelle zwischen den äußeren Kanten befindet.

10. Wiederverwendbares Menstruations- und/oder Inkontinenzkleidungsstück nach Anspruch 9, wobei das erste Ende (36) der inneren Lage (28) so geformt ist, dass an der zentralen Stelle zwischen den äußeren Kanten eine Aussparung (50) vorliegt, wobei die Aussparung (50) optional eine gekrümmte Form, optional einen Bogen, umfasst.

11. Wiederverwendbares Menstruations- und/oder Inkontinenzkleidungsstück nach einem vorhergehenden Anspruch, wobei das zweite Ende (38) der inneren Lage (28) an dem zweiten Ende (22) der äußeren Lage (12) an Taillenbefestigungszonen befestigt ist, die sich zu äußeren Kanten des zweiten Endes (38) der inneren Lage (28) hin befinden.

12. Wiederverwendbares Menstruations- und/oder Inkontinenzkleidungsstück nach Anspruch 11, wobei das zweite Ende (38) der inneren Lage (28) an dem zweiten Ende (22) der äußeren Lage (12) an einer zentralen Stelle des zweiten Endes (38) der inneren Lage (28) nicht befestigt ist, wobei sich die zentrale Stelle zwischen den äußeren Kanten befindet, optional wobei das zweite Ende (38) der inneren Lage (28) so geformt ist, dass an der zentralen Stelle zwischen den äußeren Kanten eine Aussparung (50) vorliegt, und optional wobei die Aussparung (50) eine gekrümmte Form, optional einen Bogen, umfasst.

13. Wiederverwendbares Menstruations- und/oder Inkontinenzkleidungsstück nach einem vorhergehenden Anspruch, wobei das hochelastische Gewebe durch die Elastizität des Gewebes um mindestens etwa 90 % oder mindestens etwa 100 % oder mindestens etwa 110 % oder mindestens etwa 120 % oder mindestens etwa 130 % oder mindestens etwa 140 % oder mindestens etwa 150 % gedehnt werden kann.

14. Wiederverwendbares Menstruations- und/oder Inkontinenzkleidungsstück nach einem vorhergehenden Anspruch, wobei das hochelastische Gewebe ein vierfach elastisches Gewebe umfasst und/oder wobei das hochelastische Gewebe aus Nylon, Polyester, Polyamid, Elasthan ausgewählt ist, wobei das hochelastische Gewebe optional 15-40 % Elasthan umfasst.

15. Wiederverwendbares Menstruations- und/oder Inkontinenzkleidungsstück nach einem vorhergehenden Anspruch, wobei die äußere Lage (12) nahtlos ist.

## Revendications

1. Vêtement menstruel et/ou d'incontinence réutilisable comprenant :
une feuille extérieure lavable (12) comprenant une partie de taille avant (14), une partie de taille arrière (16) et une partie d'entrejambe (18) s'étendant entre les parties de taille avant et arrière (14,16), la feuille extérieure lavable (12) présentant une première extrémité (20) au niveau de la partie de taille avant (14), une seconde extrémité (22) au niveau de la partie de taille arrière (16) et des côtés opposés (24,26) s'étendant entre les première et seconde extrémités (20,22), les parties de taille avant et arrière (14,16) étant jointes pour former des ouvertures pour la taille et les jambes, dans lequel la feuille extérieure (12) comprend un tissu hautement étirable qui est extensible par l'étirement du tissu d'au moins 80 % ;
une feuille intérieure lavable (28) comprenant une partie de taille avant intérieure (30), une partie de taille arrière intérieure (32) et une partie d'entrejambe intérieure (34) s'étendant entre les parties de taille avant et arrière intérieures (30,32), la feuille intérieure (28) présentant une première extrémité (36) au niveau de la partie de taille avant, une seconde extrémité (38) au niveau de la partie de taille arrière et des côtés opposés (40,42) s'étendant entre les première et seconde extrémités (36,38), dans lequel les parties de taille avant et arrière intérieures comprennent un tissu hautement étirable qui est extensible par l'étirement du tissu d'au moins 80 % ;
un gousset (44) comprenant une couche absorbante lavable et réutilisable conçue pour l'absorption de fluide menstruel et/ou d'urine, le gousset étant ménagé au niveau de la partie d'entrejambe intérieure (34) de la feuille intérieure (28) ;
dans lequel la feuille intérieure (28) et la feuille extérieure (12) sont reliées l'une à l'autre au niveau de leurs premières extrémités (20,36) et de leurs secondes extrémités (22,38) respectivement.

2. Vêtement menstruel et/ou d'incontinence réutilisable selon une quelconque revendication précédente, dans lequel la feuille intérieure et extérieure (28,12) sont reliées l'une à l'autre au niveau du gousset (44) au niveau d'une ou de plusieurs zones de fixation au gousset.

3. Vêtement menstruel et/ou d'incontinence réutilisable selon la revendication 2, dans lequel le gousset (44) est allongé avec des première et seconde extrémités (60,62) et des côtés opposés (64,66), dans lequel les zones de fixation sont conçues pour former des parties non fixées (76,78) au niveau de l'une ou des deux des première et seconde extrémités (60,62) du gousset (44), dans lequel le gousset (44) est non fixé à la feuille extérieure (12) au niveau des parties non fixées (76,78) ; éventuellement
dans lequel la distance entre la première extrémité (60) et la zone de fixation au niveau de la première partie non fixée de la première extrémité comprend au moins environ 1 cm, ou au moins environ 2 cm, ou au moins environ 3 cm, ou au moins environ 4 cm, ou au moins environ 5 cm, ou au moins environ 6 cm, ou au moins environ 7 cm et/ou dans lequel la distance entre la seconde extrémité (62) et la zone de fixation au niveau de la seconde extrémité de la partie non fixée comprend au moins environ 1 cm, ou au moins environ 2 cm, ou au moins environ 3 cm, ou au moins environ 4 cm, ou au moins environ 5 cm, ou au moins environ 6 cm, ou au moins environ 7 cm.

4. Vêtement menstruel et/ou d'incontinence réutilisable selon une quelconque revendication précédente, dans lequel le gousset (44) est fixé aux parties avant et arrière intérieures de la feuille intérieure (28) par tout moyen approprié, tel qu'une couture, un adhésif et une liaison thermique.

5. Vêtement menstruel et/ou d'incontinence réutilisable selon une quelconque revendication précédente, dans lequel les feuilles intérieure et extérieure (28,12) sont uniquement reliées l'une à l'autre au niveau de la taille et du gousset (44).

6. Vêtement menstruel et/ou d'incontinence réutilisable selon une quelconque revendication précédente, dans lequel les côtés (40,42) des parties avant et arrière intérieures de la feuille intérieure (28) sont non fixés à la feuille extérieure (12).

7. Vêtement menstruel et/ou d'incontinence réutilisable selon une quelconque revendication précédente, dans lequel la partie de taille avant intérieure (30) et la partie de taille arrière intérieure (32) de la feuille intérieure (28) ne sont pas jointes l'une à l'autre.

8. Vêtement menstruel et/ou d'incontinence réutilisable selon une quelconque revendication précédente, dans lequel la première extrémité (36) de la feuille intérieure (28) est fixée à la première extrémité (20) de la feuille extérieure (12) au niveau de zones de fixation à la taille, les zones de fixation à la taille étant situées vers des bords extérieurs de la première extrémité (36) de la feuille intérieure (28).

9. Vêtement menstruel et/ou d'incontinence réutilisable selon la revendication 8, dans lequel la première extrémité (36) de la feuille intérieure (28) n'est pas fixée à la première extrémité (20) de la feuille extérieure (12) à un emplacement central de la première extrémité (36) de la feuille intérieure (28), l'emplacement central étant situé entre les bords extérieurs.

10. Vêtement menstruel et/ou d'incontinence réutilisable selon la revendication 9, dans lequel la première extrémité (36) de la feuille intérieure (28) est formée de sorte qu'il existe un évidement (50) au niveau de l'emplacement central entre les bords extérieurs, éventuellement dans lequel l'évidement (50) comprend une forme incurvée, éventuellement un arc.

11. Vêtement menstruel et/ou d'incontinence réutilisable selon une quelconque revendication précédente, dans lequel la seconde extrémité (38) de la feuille intérieure (28) est fixée à la seconde extrémité (22) de la feuille extérieure (12) au niveau de zones de fixation à la taille situées vers des bords extérieurs de la seconde extrémité (38) de la feuille intérieure (28).

12. Vêtement menstruel et/ou d'incontinence réutilisable selon la revendication 11, dans lequel la seconde extrémité (38) de la feuille intérieure (28) est non fixée à la seconde extrémité (22) de la feuille extérieure (12) au niveau d'un emplacement central de la seconde extrémité (38) de la feuille intérieure (28), l'emplacement central étant situé entre les bords extérieurs, éventuellement dans lequel la seconde extrémité (38) de la feuille intérieure (28) est formée de sorte qu'il existe un évidement (50) au niveau de l'emplacement central entre les bords extérieurs, et éventuellement dans lequel l'évidement (50) comprend une forme incurvée, éventuellement un arc.

13. Vêtement menstruel et/ou d'incontinence réutilisable selon une quelconque revendication précédente, dans lequel le tissu hautement étirable peut être étendu par l'étirement de tissu d'au moins environ 90 %, ou d'au moins environ 100 %, ou d'au moins environ 110 %, ou d'au moins environ 120 %, ou d'au moins environ 130 %, ou d'au moins environ 140 %, ou d'au moins environ 150 %.

14. Vêtement menstruel et/ou d'incontinence réutilisable selon une quelconque revendication précédente, dans lequel le tissu hautement étirable comprend un tissu étirable en quatre directions et/ou dans lequel le tissu hautement étirable est choisi parmi le nylon, le polyester, le polyamide, l'élasthanne, éventuellement dans lequel le tissu hautement étirable comprend 15 à 40 % d'élasthanne.

15. Vêtement menstruel et/ou d'incontinence réutilisable selon une quelconque revendication précédente, dans lequel la feuille extérieure (12) est sans couture.
